Europäisches Patentamt

European Patent Office

Office européen des brevets

(19)

(11) Publication number : **0 147 207 B2**

(12) NEW EUROPEAN PATENT SPECIFICATION

(45) Date of publication of the new patent
specification : **10.03.93 Bulletin 93/10**

(51) Int. Cl.⁵ : **A01N 59/00,** A01N 25/22,
    // (A01N59/00, 37:16)

(21) Application number : **84309003.6**

(22) Date of filing : **21.12.84**

(54) Disinfectants.

The file contains technical information
submitted after the application was filed and
not included in this specification

(30) Priority : **22.12.83 GB 8334249**

(43) Date of publication of application :
**03.07.85 Bulletin 85/27**

(45) Publication of the grant of the patent :
**10.02.88 Bulletin 88/06**

(45) Mention of the opposition decision :
**10.03.93 Bulletin 93/10**

(84) Designated Contracting States :
**AT BE CH DE FR GB IT LI NL SE**

(56) References cited :
**DE-A- 1 443 853**
**DE-A- 2 616 049**
**GB-A- 1 560 525**
**GB-A- 1 561 680**
**US-A- 4 051 058**

(56) References cited :
**Produktdatenblatt Marlon A330 der Hüls AG**
**Produktdatenblatt Marlon AS-R der Hüls AG**
**Hücke. "über die Eigenschaften der Peressig-**
**säure" in "Wissenschaftliche Zeizung der**
**Universität Rostock" 1970. pp 267-270.**
**Sawaki et al.Chem. Society Japan, vol. 38.**
**1965.pp 2103-2106**
**Dankowski et al. "Wasserstoffperoxid" Fir-**
**mendruckschrift der Degussa AG. "Stets**
**geforscht" Band 2, 1988. S. 1,4,53,65-67, 79,**
**80.**

(73) Proprietor : **Albright & Wilson Limited**
**Albright & Wilson House Hagley Road West**
**Oldbury Warley West Midlands, B68 ONN (GB)**

(72) Inventor : **Langford, Philip Webster**
**17 Ellacombe Road**
**Longwell Green Bristol BS15 6BQ (GB)**

(74) Representative : **Savidge, Roger Gordon**
**Madgwick et al**
**c/o Albright & Wilson Limited, P.O. Box 2098,7**
**Stars Road**
**Oldbury, Warley, West Midlands B69 4PR (GB)**

EP 0 147 207 B2

## Description

This invention relates to disinfectant concentrates comprising peracetic or perpropionic acids which are suitable for dilution with water to produce solutions having microbiocidal properties. The pure per acids are not usually manufactured because they are difficult to handle and are highly combustible. They are commercially available as concentrated solutions of the peroxy acid and the carboxylic acid which typically contain 35 to 45% by weight of peracid and 40 to 55% by weight of carboxylic acid. These concentrates are highly caustic, have extremely pungent odours and require careful handling. Moreover, when the peracid solution is diluted it is unstable and deteriorates on storage.

British Patent No. 1561680 describes concentrates comprising 0.5 to 20% by weight of the per acid and/ or carboxylic acid, 25 to 40% by weight of hydrogen peroxide (expressed as anhydrous $H_2O_2$) and water. This patent also describes the addition of sulphonate or sulphate surface active agents such as alkyl benzene sulphonates to the concentrates described therein in order to improve the wetting properties of the disinfectant obtained by dilution of the concentrate and states that the addition of these surface active agents does not destabilise the concentrates to the same degree as is found when soaps or non-ionic surface active agents are added.

We have now discovered that the addition of a sulphonic acid as a wetting agent produces a concentrate which is significantly more stable upon prolonged storage. Thus from one aspect our invention provides a concentrated disinfectant composition consisting of (i) a stable mixture comprising hydrogen peroxide, peracetic acid or perpropionic acid, and acetic or propionic acid; and (z) a wetting agent characterised in that said wetting agent consists of free alkylbenzene sulphonic acid.

Any sulphonic acid which exhibits surface active properties may be useful according to the invention. Examples of such acids are the alkyl aryl sulphonic acids which comprise from 6 to 18 carbon atoms in the alkyl substituent of the molecule especially those which comprise from 9 to 15 carbon atoms in the alkyl substituent. The sulphonic acid may take the form of a mixture of alkyl aryl sulphonic acids wherein the number of carbon atoms in the alkyl substituent varies but has an average value of from 6 to 18 carbon atoms.

Examples of alkyl aryl sulphonic acids which are useful according to the present invention include acids derived from aromatic nuclei other than benzene, such as toluene and xylene, as well as those derived from benzene itself. Examples of useful sulphonic acids include decyl toluene, dodecyl xylene, octyl benzene, nonyl benzene, decyl benzene, tridecyl benzene, tetradecyl benzene, pentadecyl benzene, dodecyl benzene and hexadecyl benzene, sulphonic acids. The preferred sulphonic acid is dodecyl benzene sulphonic acid.

The preferred per acid for use in the composition of the invention is peracetic acid. For convenience the invention will hereafter be described with reference to peracetic acid, although it should be understood that the description is equally pertinent to the use of perpropionic acid.

The concentrates may be produced by mixing the components thereof in any order. The peracetic acid may be added as a solution thereof or may be generated in situ by the reaction of hydrogen peroxide with acetic acid or acetic anhydride. The product of these mixing procedures is an aqueous solution comprising hydrogen peroxide, peracetic acid and acetic acid in dynamic equilibrium. The rate of reaction between hydrogen peroxide and acetic acid is relatively slow at ambient temperatures and the mixture may not reach equilibrium for a considerable period in some circumstances. These processes represent the preferred method for the production of the concentrates and the conditions under which the mixing of the appropriate quantities of peroxide and the acetic or peracetic acid is carried out may be adjusted to ensure that a predetermined minimum concentration of peracetic acid is produced.

The concentrates of our invention may preferably comprise from 0.5 to 20% by weight of peracetic acid, preferably from 1.0 to 10.0 by weight and more preferably from 2.0 to 7.0 by weight of peracetic acid. In general the stability of the concentrates increases as the concentration of the peracetic acid increases and should be at least 3.0% by weight and more preferably at least 3.5% by weight of the concentrate. Where the concentrate is produced by the reaction of hydrogen peroxide with acetic acid a relatively initial high concentration of peracetic acid may be produced depending upon the reaction conditions and the reaction times but the concentration returns to the equilibrium value on standing at ambient temperatures.

The concentration of peroxide in the concentrates of this invention will be sufficient to stabilise the peracetic acid to the desired degree. In general, the concentration of hydrogen peroxide (expressed as anhydrous material) will be in the range 5 to 50%, preferably 10 to 35%, and most preferably 15 to 25% by weight of the concentrate. The ratio of the weight of hydrogen peroxide to the weight of peracetic acid will normally be in the range 2:1 to 10:1 preferably in the range 4:1 to 7:1. The ratio of the weight of the hydrogen peroxide to the weight of acetic acid in the concentrates of the invention is normally in the range 1.0:1 to 5.0:1 and more preferably in the range 1.5:1 to 3.5:1. Since the reaction between hydrogen peroxide and acetic acid to produce peracetic acid does not proceed to completion at ambient temperature the concentrates will always contain some acetic acid and the ratio of the concentrations of peracetic acid to acetic acid will be constant provided that the system has been allowed to reach equilibrium. Thus the ratio of the amount of peroxide to the amount of peracetic acid present in the concentrates (at equilibrium) will dictate the ratio of the amount of peroxide to the amount of acetic acid and vice versa. The choice of which ratio to adjust in order to improve the stability is dictated by convenience depending upon the manner in which the concentrates are made up.

The stability of the concentrates decreases as the amount of water present increases. The amount of water present in the concentrates is preferably in the range 40 to 75% by weight and more preferably in the range 50 to 70%. The ratio of the amount of water to the amount of peracetic acid (at equilibrium) is preferably in the range 10: 1 to 20:1 more preferably in the range 12:1 to 18:1. The maximum concentration of peracetic acid is preferably not greater than 7.0% and more preferably not greater than 5.0% by weight because greater concentrations of peracetic acid present difficulties in transporting and handling the concentrate. Where the peracetic acid is to be generated in situ by the reaction of hydrogen peroxide with acetic acid or acetic anhydride, the quantity of hydrogen peroxide which is used will be adjusted so as to take into account the quantity of hydrogen peroxide which is liable to be consumed in this reaction.

The peracetic acid containing concentrate is preferably produced by adding glacial acetic acid to a solution of hydrogen peroxide. The formulation of peracetic acid may be accelerated by the addition of a catalytic quantity of a mineral acid, but this is less preferred since it adds to the corrosive nature of the product. Alternatively the concentrate can be produced by the addition of hydrogen peroxide solution to commercially available peracetic acid products.

The disinfectant concentrates preferably further comprise a stabiliser for the peracetic acid. Commercial grades of peracetic acid may well incorporate such a stabiliser in which case there is no need to add additional quantities of stabilisers. If the concentrate is produced from acetic acid it is preferable to add the stabiliser. Compounds which are known to be useful as stabilisers for peracetic acid include 2,6-pyridine dicarboxylic acid and phytic acid.

These stabilisers are added in conventional quantities e.g. 0.0001 to 1.0% by weight of the concentrate.

The reagents used in the preparation of the concentrates should not contain any impurities which may serve to destabilise the peracetic acid. The hydrogen peroxide, the sulphonic acid; the stabiliser and the acetic acid or acetic anhydride are all preferably free from significant quantities of these impurities. The presence of a limited quantity of various impurities in the reagents may be tolerated and, generally, reagents which are sold as Technical Grade Reagents may be used in the concentrates of this invention. The use of purified grades of reagent material leads to the production of a more stable concentrate but the increase in stability is not usually sufficient to compensate for the increased expense of these reagents and the use of Technical Grade reagents is usually preferable on economic grounds.

Where water is employed in the formulation of the concentrates in addition to the water which is supplied with the other compounds, e.g. that added in the form of an aqueous solution of hydrogen peroxide, deionised water is preferably employed. The ionic impurities which may be present in tap water tend to destabilise the peracetic acid and the use of these grades of water is thereby less preferred. In general the water employed, if any, will preferably contain less than 400 ppm of dissolved solids and more preferably less than 35 ppm of dissolved solids.

The destabilising effect of these various impurities is cummulative and thus the use of relatively impure grades of the various reagents is disadvantageous since the total amount of impurities in the concentrate is increased. The presence of dissolved cations (other than hydrogen) is particularly disadvantageous and in general the concentrates will comprise less than 250 ppm and more preferably less than 50 ppm of dissolved cations.

The novel concentrates of this invention may comprise one or more sequestering agents which are capable of sequestering bivalent metal ions. The addition of appropriate quantities of such agents e.g. from 0.1 to 10% by weight of the concentrate decreases the unstability of the peracetic acid in the presence of polyvalent metal ions. One example of compounds which are useful as sequestering agents are phosphonic acids including all those which are described as being useful for this purpose in United States Patent 4051058. Examples of useful phosphonic acids are ethylene diamine tetra(methylene phosphonic acid), dimethylaminomethane diphosphonic acid, amino tri (methylene phosphonic acid) and 1-amino-1-phenylmethane diphosphonic acid.

The alkyl benzene sulphonic acid may be added at any stage during the production of the concentrate, but is preferably added after the other ingredients of the concentrate have been admixed. The sulphonic acids may normally be added in the form of an aqueous solution thereof. The amount of sulphonic acid will normally be that which confers the desired degree of wetting properties to the disinfectant produced by dilution of the concentrate. Preferably the sulphonic acid will be present in an amount of from 0.1 to 5.0% more preferably from 0.5 to 5% by weight of the concentrate. Larger quantities may be employed but this is not normally necessary and may be disadvantageous since the presence of excessive quantities of sulphonic acid may decrease the stability of the peracetic acid. The presence of from 1.0 to 2.0% by weight of the sulphonic acid is normally sufficient to provide the necessary wetting properties.

The formulated concentrates normally have a pH in the range 0.5 to 2.0.

The concentrates may further comprise conventional additives such as dyestuffs, perfumes. They should preferably not contain any ingredients which serve to destabilise the peracetic acid. Metal ions especially polyvalent metal ions are known to destabilise the acid and they are preferably excluded from the concentrate.

As hereinbefore described, the concentrates of this invention may be produced by admixture of the various ingredients in any order. However we prefer to produce them by mixing hydrogen peroxide with acetic acid or acetic anhydride and allowing the peracetic acid forming reaction to proceed substantially as far as the equilibrium concentration prior to the addition of the sulphonic acid. The reaction between the hydrogen peroxide and the acetic acid or acetic anhydride is preferably carried out using concentrated solutions of these reagents. Glacial acetic acid or acetic anhydride is preferably employed whilst the hydrogen peroxide is preferably an aqueous solution comprising at least 25% of anhydrous $H_2O_2$ and preferably at least 30%. The reaction between hydrogen peroxide and acetic acid at room temperature proceeds relatively slowly and it is preferable to allow the mixture to stand for several days in order to produce the equilibrium concentration of peracetic acid. The reaction proceeds more rapidly at temperatures above the ambient, e.g. if the mixture is maintained at a temperature of 50°C, equilibrium may be reacted within 12 to 24 hours. Prolonged exposure to these elevated temperatures produces more peracetic acid but such processes are less preferred since this concentration will drop when the mixture is allowed to cool. The reaction between hydrogen peroxide and acetic anhydride is exothermic and proceeds more rapidly at room temperatures and equilibrium may be reached within 1 to 2 hours.

The stabiliser for the peracetic acid may conveniently be added to the mixture before equilibrium is reached. The sulphonic acid is preferably added after the equilibrium has been reached in the form of an aqueous solution thereof in the quantity of water needed to produce the concentrate.

The novel concentrates of the invention are stable on prolonged storage at ambient temperatures. They retain their disinfectant activity even on prolonged storage. In use, the concentrates are diluted with water, say 50, 75, 100 or even 300 times depending upon the application for which the disinfectant is intended. The compositions may also be diluted with an aqueous solution of a primary alcohol such as industrial methylated spirits or ethanol to give a composition having an enhanced sporicidal value.

The invention is illustrated by the following Examples.

Example 1

| | Percent m/m |
|---|---|
| Acetic acid | |
| Hydrogen peroxide (35% solution) | 70.9 |
| 2:6 Pyridine dicarboxylic acid | 0.015 |
| Dodecyl benzene sulphonic acid | 1.2 |
| Water | 17.585 |

The composition was mixed, left to stand at 50°C for 19 hours, cooled to ambient temperature and samples at intervals to determine the hydrogen peroxide and peracetic acid contents with results as follows.

4

TABLE 1

| Time (after blending) | $H_2O_2$% m/m | Peracetic acid % m/m |
|---|---|---|
| 19 hours | 23.0 | 4.1 |
| 5 days | 23.0 | 4.1 |
| 12 days | 23.1 | 4.0 |
| 42 days | 23.1 | 4.0 |
| 91 days | 23.2 | 3.9 |

Example 1 was tested against Salmonella chloeraesuis (NCTC No. 10653) by the method of test for General Purpose Disinfectants detailed in Explanatory Note on the Approval of Disinfectants for the purposes of the Diseases of Animals Act 1950, published by the Ministry of Agriculture, Fisheries & Food, and passed at a dilution of 1 part disinfectant to 200 parts water.

It was also tested against the following microorganisms by the appropriate methods detailed in the same Explanatory Note with results as follows:

| Microorganism | Dilution of Example 1 passing the test |
|---|---|
| Mycobacterium fortuitum | 1—75 |
| Foot & Mouth Disease virus | 1—75 |
| Fowl Pest virus | 1—100 |
| Swine Vesicular Disease virus | 1—75 |

Example 2

The following compositions were mixed, allowed to stand at 50°C for 16 hours, cooled to ambient temperature and sampled at intervals to determine the hydrogen peroxide and peracetic acid contents. Compositions 2, 4 and 6 are examples of compositions according to this invention, whereas compositions 3 and 5 are comparative examples illustrating the relative instability of compositions containing sulphonates. The results are summarised in the following table (where PAA = Peracetic Acid).

| Example No. | 2 | 3 | 4 | 5 | 6 |
|---|---|---|---|---|---|
| Type | Perpropionic acid | Commercial dodecyl benzene sulphonate | dodecyl benzene sulphonic acid (commercial) | purified dodecyl benzene sulphonate | Alkyl benzene sulphonic acid (Commercial) without stabiliser |
| **Composition % m/m** | | | | | |
| Acetic acid | — | 12.7 | 11.3 | 11.3 | 11.3 |
| Propionic acid | 15.2 | — | — | — | — |
| Hydrogen peroxide 35.4% solution | 83.382 | 85.782 | 76.6 | 76.6 | 76.6 |
| 2:6 Pyridine dicarboxylic acid | 0.018 | 0.018 | 0.016 | 0.016 | — |
| Sodium dodecyl benzene sulphonate (Commercial — containing 20% inorganic salts) | — | 1.5 | — | — | — |
| Sodium dodecyl benzene sulphonate (Purified) | — | — | — | 1.3 | — |
| Dodecyl benzene sulphonic acid | 1.4 | — | 1.3 | — | 1.3 |
| Water | — | — | 10.784 | 10.784 | 10.8 |
| **Analysis % m/m** | | | | | |
| $H_2O_2$ after 16 Hours at 50°C | 26.6 | 28.0 | 24.6 | 24.3 | 24.6 |
| PAA after 16 Hours at 50°C | 6.3 | 1.7 | 4.5 | 3.1 | 4.5 |
| (Example 2 — Perpropionic Acid) | | | | | |
| $H_2O_2$ after (x) days (Example 2 PPA) | 26.0 (35) | 22.8 (35) | 24.4 (35) | 22.8 (42) | 23.9 (30) |
| PAA after (x) days (Example 2 PPA) | 6.9 (35) | 2.2 (35) | 4.3 (35) | 3.9 (42) | 4.2 (30) |
| $H_2O_2$ after (x) days | 25.9 (84) | 18.2 (84) | 24.3 (80) | — | 23.1 (79) |
| PAA after (x) days | 7.4 (84) | 1.6 (84) | 4.3 (80) | — | 4.1 (79) |
| $H_2O_2$ after (x) days | 25.5 (194) | 13.2 (194) | 24.0 (190) | 20.4 (152) | 21.5 (190) |
| PAA after (x) days | 7.1 (194) | 1.4 (194) | 4.2 (190) | 3.65 (152) | 3.9 (190) |

Example 3

A series of formulations A, B and C were prepared by blending components 1-3 and holding at 55°C for 17 hours followed by addition of remaining components.

6

| RM % m/m Example | A | B | C |
|---|---|---|---|
| 1) Glacial Acetic Acid | 10.3 | 10.3 | 10.3 |
| 2) Hydrogen peroxide 34.9% | 70.9 | 70.9 | 70.9 |
| 3) 2:6 Pyridine dicarboxylic acid | 0.015 | 0.015 | 0.015 |
| 4) Dodecyl benzene sulphonic acid | 1.2 | — | — |
| 5) Sodium dodecyl benzene sulphonate | — | 1.2 | — |
| 6) Sodium lauryl sulphate (pure) | — | — | 1.2 |
| 7) Sulphuric acid | — | 0.11 | 0.09 |
| 8) Soft water | 17.585 | 17.475 | 17.495 |
| Analysis | | | |
| $H_2O_2$ % | 23.1 (3) | 23.1 (3) | 23.1 (3) |
| (after x days) | 23.0 (11) | 22.8 (11) | 22.9 (11) |
| PAA % | 3.8 (3) | 3.4 (3) | 3.4 (3) |
| (after x days) | 4.0 (11) | 3.6 (11) | 3.8 (11) |
| pH after 11 days | 1.3 | 1.3 | 1.3 |

This example illustrates that the dodecyl benzene sulphonic acid containing solution is more stable than solutions containing sodium dodecyl benzene sulphonate or sodium lauryl sulphate at identical pH values.

Example 4

The following compositions according to the invention were mixed, allowed to stand at 50°C for 16 hours and sampled at intervals to determine the hydrogen peroxide and peracetic acid contents. The results are presented in the following table:-

| R.M. % m/m | A | A1 | A2 | A3 | A4 | D2 |
|---|---|---|---|---|---|---|
| Glacial Acetic Acid | 10.3 | 9.8 | 10.5 | 10.2 | 11.3 | — |
| Propionic Acid | — | — | — | — | — | 15.2 |
| (35.4%) H$_2$O$_2$ | — | — | — | — | — | 83.382 |
| (34.9%) H$_2$O$_2$ | 70.9 | 70 | 71 | 69.1 | 76.6 | — |
| 2:6 Pyridine dicarboxylic acid | 0.015 | 0.014 | 0.015 | 0.015 | 0.016 | 0.018 |
| Dodecyl benzene sulphonic acid* | 1.2 | 1.14 | 1.2 | 1.2 | 1.3 | 1.4 |
| Soft Water added x days later | 17.585 | 19.046 | 17.285 | 19.485 | 10.784 | — |

**ANALYSIS**

| | A | A1 | A2 | A3 | A4 | D2 |
|---|---|---|---|---|---|---|
| H$_2$O$_2$ % after 16 hours at 50°C | — | 23.1 | 22.9 | 27.4 | 27.5 | 26.6 |
| Peracetic Acid% or Perpropionic Acid % (P) after 16 hours at 50°C | — | 3.6 | 3.8 | 5.5 | 5.9 | 6.3 (P) |
| H$_2$O$_2$ % after (y) days | 23.0 (5) | 23.0 (4) | 23 (13) | 22.6 (3) | 24.6 (2) | 26.5 (3) |
| | 23.1 (42) | 22.9 (9) | 23.4 (34) | 22.5 (11) | 24.6 (6) | 26.5 (11) |
| | | 22.7 (40) | | 22.4 (35) | 24.4 (30) | 26.0 (35) |
| | 21.1 (574) | 22.0 (384) | 21.5 (378) | 21.6 (379) | 23.4 (373) | 24.4 (377) |
| Peracetic Acid % or Perpropionic Acid % (P) after y days | 4.1 (5) | 3.6 (4) | 3.8 (13) | 3.8 (3) | 4.5 (2) | 7.1 (P) (3) |
| | 4.0 | 3.6 (9) | 3.9 | 3.4 | 4.3 | (11) |
| | 3.5 (42) | 3.7 | 3.6 (34) | 3.6 (35) | 4.2 (30) | 6.9 (P) (35) |
| | (574) | (384) | (378) | (379) | (373) | 6.5 (P) (377) |
| Patent Example Number | 1 | — | — | — | 4 | 2 |

\* as sold under the Trade Mark NANSA 1042P by Albright & Wilson Limited

## Claims

1. A concentrated disinfectant composition consisting of : (1) a stable mixture comprising hydrogen peroxide, peracetic or perpropionic acid, and acetic or propionic acid; and (2) a wetting agent,
   characterised in that
   said wetting agent consists of free alkyl benzene sulphonic acid.

2. A composition according to claim 1, characterised in that, the sulphonic acid comprises from 6 to 18 carbon atoms in the alkyl substituent of the molecule.

3. A composition according to claim 2, characterised in that, the alkyl substituted aryl sulphonic acid comprises from 9 to 15 carbon atoms in the alkyl substituent of the molecule.

4. A composition according to either of claims 2 or 3 characterised in that, alkyl benzene sulphonic acid is selected from decyl toluene sulphonic acid; dodecyl xylene sulphonic acid, octyl benzene sulphonic acid, nonyl benzene sulphonic acid, decyl benzene sulphonic acid, tridecyl benzene sulphonic acid, tetradecyl benzene sulphonic acid, pentadecyl benzene sulphonic acid, dodecyl benzene sulphonic acid, and hexadecyl benzene sulphonic acid.

5. A composition according to claim 4, characterised in that, wherein alkyl benzene sulphonic acid is dodecyl benzene sulphonic acid.

6. A composition according to any of claims 1 to 5, characterised in that, it comprises from 0.1 to 5.0% by weight of sulphonic acid.

7. A composition according to claim 6, characterised in that it comprises from 1.0 to 2.0% of sulphonic acid.

8. A composition according to any of the preceding claims, characterised in that it comprises from 0.5 to 20.0% by weight of peracetic or perpropionic acid.

9. A composition according to claim 8, characterised in that it comprises from 1.0 to 10% by weight of peracetic or perpropionic acid.

10. A composition according to claim 9 characterised in that it comprises from 2.0 to 7.0% of peracetic or prepropionic acid.

11. A composition according to any of the preceding claims, characterised in that it comprises from 5 to 50% by weight of hydrogen peroxide.

12. A composition according to claim 11, comprises from 10 to 35% by weight of hydrogen peroxide.

13. A composition according to any of the preceding claims, characterised in that the ratio of the weight of hydrogen peroxide to the weight of peracetic acid is in the range 2:1 to 10:1.

14. A composition according to any of the preceding claims, characterised in that it comprises from 40 to 75% by weight of water.

15. A composition according to claim 14 characterised in that it comprises from 50 to 70% by weight of water.

16. A composition according to any of the preceding claims, characterised in that the concentrate comprises less than 250 ppm of dissolved cations (other than hydrogen).

17. A composition according to any of the preceding claims which comprises a stabiliser for the peracetic acid or perpropionic acid.

18. A composition according to claim 17, characterised in that the stabiliser is selected from a group comprising 2,6-pyridine dicarboxylic acid and phytic acid.

19. A composition according to either of claims 17 or 18, which comprises from 0.0001 to 1.0% by weight of the stabiliser.

**Patentansprüche**

1. Konzentrierte, desinfizierende Zusammensetzung, bestehend aus: (1) einer stabilen Mischung, in der Wasserstoffperoxid, Peressigsäure oder Perpropionsäure und Essigsäure oder Propionsäure enthalten sind; und (2) einem Netzmittel, **dadurch gekennzeichnet, daß** das Netzmittel aus freier Alkylbenzolsulfonsäure besteht.

2. Zusammensetzung nach Anspruch 1, **dadurch gekennzeichnet, daß** die Sulfonsäure im Alkylsubstituent

9

des Moleküls 6 bis 18 Kohlenstoffatome enthält.

3. Zusammensetzung nach Anspruch 2, **dadurch gekennzeichnet, daß** die alkylsubstituierte Arylsulfonsäure im Alkylsubstituenten des Moleküls 9 bis 15 Kohlenstoffatome enthält.

4. Zusammensetzung nach einem der Ansprüche 2 oder 3, **dadurch gekennzeichnet, daß** die Alkylbenzolsulfonsäure ausgewählt ist aus Decyltoluolsulfonsäure, Dodecylxylolsulfonsäure, octylbenzolsulfonsäure, Nonylbenzolsulfonsäure, Decylbenzolsulfonsäure, Tridecylbenzolsulfonsäure, Tetradecylbenzolsulfonsäure, Pentadecylbenzolsulfonsäure, Dodecylbenzolsulfonsäure und Hexydecylbenzolsulfonsäure.

5. Zusammensetzung nach Anspruch 4, **dadurch gekennzeichnet, daß** die Alkylbenzolsulfonsäure aus Dodecylbenzolsulfonsäure besteht.

6. Zusammensetzung nach irgendeinem der Ansprüche 1 bis 5, **dadurch gekennzeichnet**, daß sie 0,1 bis 5,0 Gew.% der Sulfonsäure enthält.

7. Zusammensetzung nach Anspruch 6, **dadurch gekennzeichnet, daß** die 1,0 bis 2,0 % der Sulfonsäure enthält.

8. Zusammensetzung nach irgendeinem der vorhergehenden Ansprüche, **dadurch gekennzeichnet, daß** sie 0,5 bis 20,0 Gew.% Peressig- oder Perpropionsäure enthält.

9. Zusammensetzung nach Anspruch 8, **dadurch gekennzeichnet, daß** sie 1,0 bis 10 Gew.% Peressig- oder Perpropionsäure enthält.

10. Zusammensetzung nach Anspruch 9, **dadurch gekennzeichnet, daß** sie 2,0 bis 7,0 % Peressig- oder Perpropionsäure enthält.

11. Zusammensetzung nach irgendeinem der vorhergehenden Ansprüche, **dadurch gekennzeichnet, daß** sie 5 bis 50 Gew.% Wasserstoffperoxid enthält.

12. Zusammensetzung nach Anspruch 11, **dadurch gekennzeichnet, daß** sie 10 bis 35 Gew.% Wasserstoffperoxid enthält.

13. Zusammensetzung nach irgendeinem der vorhergehenden Ansprüche, **dadurch gekennzeichnet, daß** das Gewichtsverhältnis von Wasserstoffperoxid zu Peressigsäure im Bereich von 2 : 1 bis 10 : 1 liegt.

14. Zusammensetzung nach irgendeinem der vorhergehenden Ansprüche, **dadurch gekennzeichnet, daß** sie 40 bis 75 Gew.% Wasser enthält.

15. Zusammensetzung nach Anspruch 14, **dadurch gekennzeichnet, daß** sie 50 bis 70 Gew.% Wasser enthält.

16. Zusammensetzung nach irgendeinem der vorhergehenden Ansprüche, **dadurch gekennzeichnet, daß** das Konzentrat weniger als 250 ppm gelöster Kationen (die von Wasserstoff verschieden sind) enthält.

17. Zusammensetzung nach irgendeinem der vorhergehenden Ansprüche, die einen Stabilisator für die Peressig- oder die Perpropionsäure enthält.

18. Zusammensetzung nach Anspruch 17, **dadurch gekennzeichnet, daß** der Stabilisator aus einer Gruppe ausgewählt wird, die 2,6-Pyridindicarbonsäure und Phytinsäure enthält.

19. Zusammensetzung nach einem der Ansprüche 17 oder 18, die 0,0001 bis 1,0 Gew.% des Stabilisators enthält.

## Revendications

1. Composition désinfectante concentrée consistant en : (1) un mélange stable comprenant du peroxyde d'hydrogène, de l'acide peracétique ou de l'acide perpropionique, et de l'acide acétique ou de l'acide pro- pionique, et (2) un agent mouillant, caractérisée en ce que ledit agent mouillant consiste en un acide alkylbenzènesulfonique libre.

2. Composition selon la revendication 1, caractérisée en ce que l'acide sulfonique comprend de 16 à 18 atomes de carbone dans le substituant alkyle de la molécule.

3. Composition selon la revendication 2, caractérisée en ce que l'acide arylsulfonique substitué par un radical alkyle comprend de 9 à 15 atomes de carbone dans le substituant allyle de la molécule.

4. Composition selon l'une ou l'autre des revendications 2 ou 3, caractérisée en ce que l'acide alkylbenzènesulfonique est choisi entre l'acide décyltoluènesulfonique, l'acide dodécylxylènesulfonique, l'acide octylbenzènesulfonique, l'acide nonylbenzènesulfonique, l'acide décylbenzènesulfonique, l'acide tridécylbenzènesulfonique, l'acide tétradécylbenzènesulfonique, l'acide pentadécylbenzènesulfonique, l'acide dodécylbenzènesulfonique et l'acide hexadécylbenzènesulfonique.

5. Composition selon la revendication 4, caractérisée en ce que l'acide alkylbenzènesulfonique est l'acide dodécylbenzènesulfonique.

6. Composition selon l'une quelconque des revendications 1 à 5, caractérisée en ce qu'elle comprend de 0,1 à 5,0 % en poids d'acide sulfonique.

7. Composition selon la revendication 6, caractérisée en ce qu'elle comprend de 1,0 à 2,0 % d'acide sulfonique.

8. Composition selon l'une quelconque des revendications précédentes, caractérisée en ce qu'elle comprend de 0,5 à 20,0 % en poids d'acide peracétique ou perpropionique.

9. Composition selon la revendication 8, caractérisée en ce qu'elle comprend de 1,0 à 10 % en poids d'acide peracétique ou perpropionique.

10. Composition selon la revendication 9, caractérisée en ce qu'elle comprend de 2,0 à 7,0 % d'acide peracétique ou perpropionique.

11. Composition selon l'une quelconque des revendications précédentes, caractérisée en ce qu'elle comprend de 5 à 50 % en poids de peroxyde d'hydrogène.

12. Composition selon la revendication 11, caractérisée en ce pou'elle comprend de 10 à 35 % en poids de peroxyde d'hydrogène.

13. Composition selon l'une quelconque des revendications précédentes, caractérisée en ce que le rapport pondéral entre le peroxyde d'hydrogène et l'acide peracétique est dans l'intervalle de 2:1 à 10:1.

14. Composition selon l'une quelconque des revendications précédentes, caractérisée en ce qu'elle comprend de 40 à 75 % en poids d'eau.

15. Composition selon la revendication 14, caractérisée en ce qu'elle comprend de 50 à 70 % en poids d'eau .

16. Composition selon l'une quelconque des revendications précédentes, caractérisée en ce que le concentré comprend moins de 250 ppm de cations dissous (autres que l'hydrogène).

17. Composition selon l'une quelconque des revendications précédentes, qui comprend un stabilisant de l'acide peracétique ou de l'acide perpropionique.

18. Composition selon la revendication 17, caractérisée en ce que le stabilisant est choisi dans le groupe comprenant l'acide 2,6-pyridinedicarboxylique et l'acide phytique.

19. Composition selon l'une ou l'autre des revendications 17 ou 18, qui comprend de 0,0001 à 1,0 % en poids du stabilisant.